(19) Europäisches Patentamt
European Patent Office
Office européen des brevets

(11) EP 2 053 050 B1

(12) EUROPÄISCHE PATENTSCHRIFT

(45) Veröffentlichungstag und Bekanntmachung des Hinweises auf die Patenterteilung:
**31.08.2011 Patentblatt 2011/35**

(51) Int Cl.:
***C07D 407/10*** (2006.01) ***A61K 31/353*** (2006.01)
***A61P 25/00*** (2006.01)

(21) Anmeldenummer: **07020728.7**

(22) Anmeldetag: **23.10.2007**

(54) **Aspalathin-ähnliches Dihydrochalcon und Verfahren zur Herstellung**

Dihydrochalcone similar to aspalathin and method for its manufacture

Dihydronchalcon similaire à l'aspalathine et procédé de fabrication

(84) Benannte Vertragsstaaten:
**AT BE BG CH CY CZ DE DK EE ES FI FR GB GR HU IE IS IT LI LT LU LV MC MT NL PL PT RO SE SI SK TR**

(43) Veröffentlichungstag der Anmeldung:
**29.04.2009 Patentblatt 2009/18**

(73) Patentinhaber: **Kneipp-Werke Kneipp-Mittel-Zentrale GmbH & Co. KG
97082 Würzburg (DE)**

(72) Erfinder:
- **Frank, Bruno. Dr
97271 Kleinrinderfeld (DE)**
- **Dimpfel, Wilfried. Prof. Dr
35578 Wetzlar (DE)**

(74) Vertreter: **Keller, Günter et al
Lederer & Keller
Patentanwälte
Unsöldstrasse 2
80538 München (DE)**

(56) Entgegenhaltungen:
**DE-A1-102005 004 438**

- **H. SCHULZ ET AL.: "Quantification of quality parameters for reliable evaluation of green rooibos (Aspalathus linearis)" EUR. FOOD RES. TECHNOL., Bd. 216, 2003, Seiten 539-543, XP002462520**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 053 050 B1

Printed by Jouve, 75001 PARIS (FR)

## Beschreibung

Die vorliegende Erfindung betrifft eine neue chemische Verbindung der Formel I sowie deren pharmazeutisch annehmbare Salze und Ester. Zudem betrifft die Erfindung auch ein Verfahren zur Isolierung der Verbindung gemäß Formel I aus Rotbusch-Rohware. Die vorliegende Erfindung betrifft ebenfalls einen Rotbuschextrakt, der einen Gehalt an der Verbindung gemäß Formel I von mindestens 0,4 Gew.-% aufweist. Desweiteren betrifft die Erfindung die Verwendung der chemischen Verbindung der Formel I sowie deren pharmazeutisch annehmbare Salze und Ester sowie des erfindungsgemäßen Rotbuschextrakts als Medikament, insbesondere zur Behandlung von neurologischen und psychiatrischen Störungen des zentralen Nervensystems. Der Ausdruck pharmazeutisch wirksam umfasst auch solche Wirkungen, die zu einer subjektiven Verbesserung der Befindlichkeit führen, wobei eine arzneimittelrechtliche Zulassung nicht unbedingt erforderlich sein muss.

Der Rotbusch (englisch: Rooibos; lateinisch: Aspalathus lineares) wächst ausschließlich in Südafrika und enthält derzeit als weltweit einzige bekannte Pflanze die besonders stark antioxidativ wirkende Substanz Aspalathin, ein Flavonoid. Zudem enthält der Rotbusch weitere Flavonoide, wie C-Glycosylflavone (unter anderem Orientin, Isoorientin), Flavonol-3-O-Glycoside (unter anderem Quercetin, Quercitrin, Isoquercitrin, Rutin) und C-Glucoside der Dihydrochalcone Nothofagin und Aspalathin.

Unfermentierter "grüner" Rotbusch zeichnet sich durch einen höheren Gehalt an Polyphenolen, insbesondere Aspalathin, sowie einer höheren antioxidativen Aktivität im Vergleich zum fermentierten Produkt aus. Der Effekt der Abnahme der antioxidativen Aktivität durch den Fermentationsprozess ist gleichermaßen beim schwarzen gegenüber dem grünen Tee zu beobachten (Bramati et al., J. Agric. Food Chem. 2003, 51: 7472-7474). Wissenschaftliche Untersuchungen haben gezeigt, dass die antioxidative Aktivität des Rotbuschtees hauptsächlich auf den Aspalathingehalt zurückzuführen ist. Bei der Untersuchung des Fermentationsprozesses von Rotbuschtee wurde festgestellt, dass der Gehalt an Aspalathin und Nothofagin während des Fermentationsprozesses abnimmt (Schulz et al., Eur. Food Res. Technol. 2003, 216: 539-543). Die geringere antioxidative Aktivität von fermentiertem "rotem" Rotbuschtee gegenüber unfermentiertem "grünem" Rotbuschtee kann somit erklärt werden.

Aufgrund der vorstehend genannten gesundheitsfördernden Flavonoide sowie seinem guten Geschmack ist der Rotbuschtee weit verbreitet. Weitere Inhaltsstoffe des Rotbuschtees sind Phenolsäuren, ätherische Öle, Vitamin C sowie zahlreiche Mineralstoffe, insbesondere Eisen.

Um eine möglichst hohe antioxidative Aktivität zu erzielen, ist ein hoher Gehalt an Aspalathin notwendig. Diesbezüglich offenbart DE 10 2005 004 438 einen Rotbuschextrakt, der im Vergleich zu dem üblichen Aspalathingehalt von 1 bis 3 Gew.-% einen erhöhten Gehalt von mehr als 5 Gew.-%, bei gleichzeitig niedrigem Chlorophyllgehalt von weniger als 0,4 Gew.-%, aufweist. Gemäß DE 10 2005 004 438 wird der Rotbuschextrakt durch Extraktion aus unfermentierter Rotbusch-Rohware unter Verwendung eines Gemischs aus Ethanol und Wasser erhalten. Der Rotbuschextrakt mit hohem Aspalathingehalt soll aufgrund der stark antioxidativen, antiirritativen und antimikrobiellen Wirkung insbesondere für die kosmetische Anwendung beispielsweise als Haar-, Haut- oder Mundpflegemittel verwendet werden.

Ein weiteres Flavonoid, das in dem Rotbuschtee enthalten ist, ist Quercetin. Dies kommt mit einem Gehalt von ca. 11 mg/100 g Rotbusch-Rohmaterial vor und wirkt sich z.B. auf die Histaminfreisetzung im menschlichen Körper aus, wodurch allergische Symptome gelindert werden können. Quercetin vermag ebenso die Produktion der Monoaminooxidase zu hemmen, was leichte Depressionen und Einschlafstörungen günstig beeinflusst (Plantextrakt, the nature network, Ausgabe 3 vom 09.11.2005; Plantextrakt GmbH).

Ein Ausgangspunkt der vorliegenden Erfindung war die Suche nach Wirkstoffen zur Behandlung von Krankheiten des zentralen Nervensystems, wie beispielsweise Demenzen, Morbus Parkinson, Depressionen und Schmerzzustände. Diese Krankheiten sind schwer zu therapieren, und die hierbei eingesetzten Medikamente wie beispielsweise Tacrin, Galantamin oder Nefopam weisen ein breites Nebenwirkungsspektrum auf.

Eine Aufgabe der Erfindung ist es daher, Wirkstoffe und Zusammensetzungen zur therapeutischen Behandlung dieser Krankheiten zur Verfügung zu stellen. Insbesondere sollen diese Wirkstoffe oder Zusammensetzungen geringe Nebenwirkungen aufweisen.

Diese Aufgabe wird durch den Gegenstand der Patentansprüche gelöst.

Die vorliegende Erfindung betrifft daher eine Verbindung der Formel I

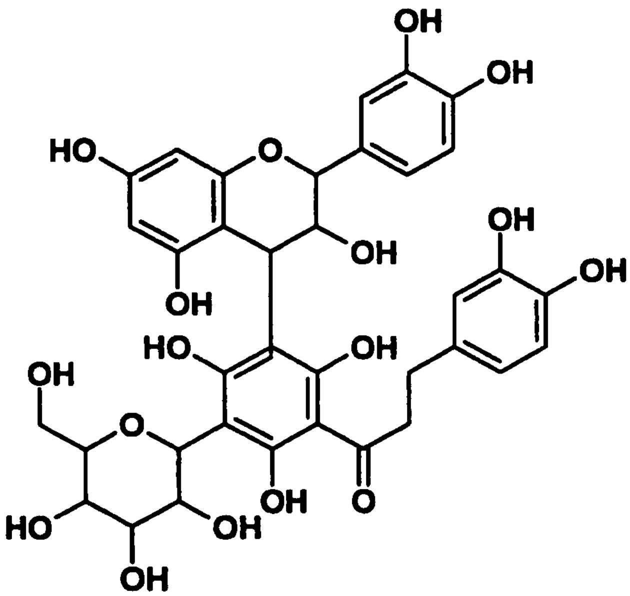

sowie deren pharmazeutisch annehmbare Salze und Ester. Derivate hiervon sind hierbei Kopplungsprodukte der Verbindung gemäß Formel I an Ferulasäure, Chinasäure, Kaffeesäure, Gluconsäure oder Chlorogensäure. Bevorzugt wird die Verbindung der Formel I in ihrer natürlichen Form oder als Salz eingesetzt, weiter bevorzugt in ihrer natürlichen Form gemäß Formel I.

Desweiteren betrifft die Erfindung einen Rotbuschextrakt, bevorzugt aus unfermentiertem Rotbusch, mit einem Gehalt an der Verbindung gemäß Formel I von mindestens 0,4 Gew.-%, weiter bevorzugt mindestens 0,8 Gew.-%, weiter bevorzugt mindestens 1,5 Gew.-%, weiter bevorzugt mindestens 5 Gew.-%, weiter bevorzugt mindestens 10 Gew.% und am meisten bevorzugt mindestens 20 Gew.-%.

Desweiteren betrifft die Erfindung ein Verfahren zur Herstellung der Verbindung der Formel I, dadurch gekennzeichnet, dass

- getrocknete und zerkleinerte, unfermentierte Rotbusch-Rohware mit einem Extraktionsmittel bestehend aus Methanol und/oder Wasser für eine vorbestimmte Extraktionsdauer bei einer Temperatur von bis zu 60°C extrahiert wird,
- das Extrakt filtriert und anschließend unter reduziertem Druck zur Trockne eingeengt wird, und anschließend
- durch drei chromatographische Trennungsschritte unter Verwendung von zwei Sephadex LH20-Säulen und anschließend einer lipophilen c18-HPLC-Säule aufgereinigt wird.

Die Erfindung betrifft auch die Verwendung eines Rotbuschextrakts als Medikament oder Nahrungsergänzungsmittel. Die Erfindung betrifft zudem die Verwendung von Verbindungen gemäß Formel I sowie deren pharmazeutisch annehmbare Salze und Ester als Medikament oder Nahrungsergänzungsmittel. Bevorzugterweise enthält das Nahrungsergänzungsmittel oder Medikament den Rotbuschextrakt in einer Menge von mindestens 100 mg, weiter bevorzugt von mindestens 200 mg, noch weiter bevorzugt von mindestens 300 mg pro g Nahrungsergänzungsmittel bzw. pro Dosierungseinheit des Medikaments. In einer weiteren bevorzugten Ausführungsform enthält das Nahrungsergänzungsmittel oder Medikament mindestens 1 mg, weiter bevorzugt mindestens 2 mg, noch weiter bevorzugt mindestens 3 mg, noch weiter bevorzugt mindestens 5 mg und am meisten bevorzugt mindestens 10 mg der Verbindung der Formel I pro g Nahrungsergänzungsmittel bzw. pro Dosierungseinheit eines Medikaments.

In einer bevorzugten Ausführungsform der Erfindung wird der Rotbuschextrakt mit dem erhöhten Anteil an der Verbindung gemäß Formel 1 zur Behandlung von neurologischen und psychiatrischen Störungen des zentralen Nervensystems, bevorzugt zur Behandlung von Demenzen, Morbus Parkinson, Depressionen und Schmerzzuständen, weiter bevorzugt Alzheimerschen Erkrankung, verwendet.

In einer weiteren bevorzugten Ausführungsform der Erfindung werden Verbindungen gemäß Formel I sowie deren pharmazeutisch annehmbare Salze und Ester zur Behandlung von neurologischen und psychiatrischen Störungen des zentralen Nervensystems, bevorzugt zur Behandlung von Demenzen, Morbus Parkinson, Depressionen und Schmerzzuständen, weiter bevorzugt Alzheimerschen Erkrankung, verwendet.

In einer noch weiter bevorzugten Ausführungsform betrifft die Erfindung die Verwendung der Verbindung der Formel I oder deren pharmazeutisch annehmbare Salze und Ester zur Herstellung eines Medikaments zur Behandlung

von neurologischen und psychiatrischen Störungen des zentralen Nervensystems, worin die neurologischen und psychiatrischen Störungen des zentralen Nervensystems Demenzen, Morbus Parkinson, Depressionen und Schmerzzustände, weiter bevorzugt Alzheimer, sind.

Im Rahmen der vorliegenden Erfindung konnte ein neuer, pharmakologisch wirksamer Naturstoff aus einem Rotbuschextrakt isoliert und anschließend charakterisiert werden. Unerwarteterweise konnte die neue chemische Verbindung nur aus unfermentiertem "grünem" Rotbuschextrakt isoliert werden. Erst die vollständige Charakterisierung der Verbindung ermöglichte den Nachweis geringster Mengen auch in dem fermentierten Rotbuschextrakt.

Das Verfahren zur Isolierung der neuen chemischen Verbindung ist in Beispiel 1 ausführlich beschrieben, die Strukturformel ist in Formel I angegeben. Die Verbindung der Formel I weist sowohl Ähnlichkeiten zur Struktur des Aspalathins als auch des Catechin(4$\alpha$->2)-phloroglucinols (Figur 1) auf. Die genaue Charakterisierung sowie die Strukturaufklärung ist ausführlich in Beispiel 2 beschrieben. Im Vergleich zu den bislang bekannten Flavonoiden weist die neue Verbindung gemäß Formel I ein hohes Molekulargewicht von 740,66 g/mol auf. Derartig hochmolekulare Naturstoffe werden üblicherweise nicht für das Wirkstoffscreening verwendet, da diese aufgrund ihres Molekulargewichts die Blut-HirnSchranke schlecht passieren können. Für den Wirkort Gehirn bzw. zur Behandlung von Krankheiten des zentralen Nervensystems werden derartige Stoffe daher eher als ungeeignet angesehen.

Unerwarteterweise zeigte jedoch eine "bias"-freie Untersuchung im Tele-Stereo-EEG (Elektroenzephalografie) der Ratte eine ausgeprägte, zentralnervöse, pharmakologische Aktivität der Verbindung gemäß Formel I. Die pharmakologischen Untersuchungen zeigten überraschenderweise, dass die Aktivität im Model Tele-Stereo-EEG bei Ratten dosisabhängige Veränderungen der EEG-Frequenzen bewirkt, wie sie nach Gabe klassischer Medikamente zur Behandlung von Demenzen (beispielsweise Galantamin oder Tacrin), Morbus Parkinson (L-DOPA), sowie Schmerzzuständen (beispielsweise Nefopam) bekannt sind.

Die pharmakologische Aktivität der erfindungsgemäßen Verbindung gemäß Formel I wurde im Rahmen der vorliegenden Erfindung mit den bekannten Inhaltsstoffen des Rotbuschextrakts wie Aspalathin, Catechin oder (-)-Epicatechin verglichen. Die experimentellen Untersuchungen sind in Beispiel 3 ausführlich beschrieben und zeigen unerwarteterweise, dass die Wirkung der Verbindung gemäß Formel I nicht mit etwa äquimolaren Mengen an Aspalathin, Catechin oder (-)-Epicatechin erreicht werden kann, obwohl die Verbindung gemäß Formel I strukturelle Ähnlichkeiten zu diesen Naturstoffen aufweist. Die neue erfindungsgemäße Verbindung gemäß Formel I besitzt eine höhere pharmakologische Aktivität als diese bekannten Inhaltsstoffe des Rotbuschextrakts und ist daher besonders geeignet für die Verwendung als Medikament.

Die Isolierung dieser neuen Verbindung mit vorteilhaften pharmakologischen Aktivitäten ermöglicht insbesondere die Herstellung eines Medikaments auf Basis der Verbindung gemäß Formel I sowohl als Monopräparat als auch in Kombination mit weiteren Wirkstoffen. Diese weiteren Wirkstoffe können in dieselbe Richtung wirken oder auch völlig andere, das Krankheitsbild auf andere Weise günstig beeinflussende Eigenschaften haben. Hierfür geeignet ist auch die Kombination dieser Verbindung mit anderen in Rotbusch enthaltenen Flavonoiden und Inhaltsstoffen im Gesamtverbund. Insbesondere sind hier Inhaltsstoffe geeignet, die eine antioxidative Wirkung aufweisen.

Eine Diskriminanzanalyse der in vivo-Daten der Verbindung gemäß Formel I zeigte, wie oben erwähnt, eine Verwandtschaft zu den Medikamenten zur Behandlung von Demenzen, Morbus Parkinson, Depression und Schmerzzuständen. Da diese Medikamente ein breites Nebenwirkungsspektrum aufweisen, ist die Verwendung der Verbindung gemäß Formel I als Medikament vorteilhaft, da Naturstoffe üblicherweise eine geringere Nebenwirkungsrate erwarten lassen. Unerwarteterweise überschreitet die neue Substanz offensichtlich zudem die Blut-Hirnschranke. Dies ist nicht allgemein üblich für Flavonoide.

Desweiteren wird ein Herstellungsverfahren für Rotbuschextrakte sowie für die neu identifizierte pharmakologisch wirksame Verbindung zur Verfügung gestellt. Durch das erfindungsgemäße Verfahren ist es möglich, einen besonders geeigneten Pflanzenextrakt zur Behandlung der vorstehend genannten Krankheiten zur Verfügung zu stellen.

Der erfindungsgemäß hergestellte Rotbuschextrakt weist einen Gehalt an der Verbindung gemäß Formel I von mindestens 1 Gew.-%, weiter bevorzugt mindestens 1,5 Gew.-%, noch weiter bevorzugt mindestens 2 Gew.-%, weiter bevorzugt mindestens 2,5 Gew.-%, noch weiter bevorzugt mindestens 3 Gew.-% und am meisten bevorzugt mindestens 5 Gew.-%, auf.

Ein erfindungsgemäßes Verfahren zur Herstellung der Verbindung gemäß Formel I weist folgende Schritte auf (siehe auch Beispiel 1):

- Bereitstellen getrockneter und zerkleinerter, unfermentierter Rotbusch-Rohware,
- Extrahieren der bereitgestellten Rohware mit einem Extraktionsmittel bestehend aus einer Mischung aus einem Alkohol, bevorzugterweise Methanol, und/oder Wasser für eine vorbestimmte Extraktionsdauer bei einer Temperatur von bis zu 60°C,
- Filtrieren des Extrakts,
- Einengen des filtrierten Extrakts unter reduziertem Druck,
- Reinigen des Extrakts in drei Schritten:

- grobe Reinigung durch Chromatographie an einer Sephadex LH20-Säule
- Feinreinigung durch Chromatographie an einer weiteren Sephadex LH20-Säule
- Auftrennung an einer lipophilen c18-HPLC-Säule.

Wenn Präparate mit erhöhtem Gehalt an Verbindungen gemäß Formel I gewünscht sind, kann der Extrakt auch nur mit einer Chromatographiesäule aufgetrennt werden.

Bevorzugterweise ist der Feuchtegehalt der bereitgestellten Rotbusch-Rohware 4% oder kleiner, da auf diese Weise eine Autofermentation des Ausgangsmaterials verhindert wird.

Als Extraktionsmittel wird gemäß einer bevorzugten Ausgestaltung des erfindungsgemäßen Verfahrens eine AlkohollWasser-Mischung im Verhältnis 50:50 bis 80:20 verwendet, je nach verwendetem Alkohol (Methanol, Ethanol, Propanol, Propan-2-ol sind geeignet). Bevorzugterweise wird eine Methanol/Wasser-Mischung von 50:50 verwendet. Bei dieser bevorzugten Ausführungsform des erfindungsgemäßen Verfahrens beträgt das Verhältnis von Rohware zu Extraktionsmittel vorzugsweise ca. 1:6, und der Schritt des Extrahierens erfolgt vorzugsweise bei erhöhter Temperatur (über 40˚C) 1 Stunde lang, ist aber auch bei Raumtemperatur und einer Dauer von beispielsweise 2 bis 5 Stunden möglich.

Das Einengen des filtrierten Extrakts erfolgt vorzugsweise bei einem Druck von weniger als 300 mbar. Die Temperatur beträgt bei dem Einengungsschritt vorzugsweise maximal 40˚C.

Die Herstellung des erfindungsgemäßen Rotbuschextrakts erfolgt nach dem vorstehend beschriebenen Verfahren, wobei das Rotbuschextrakt nach dem Einengen bis zur Trockne (ohne nachfolgende chromatographische Reinigung) erhalten wird.

Das Messverfahren zur Bestimmung des Gehalts des Rotbuschextrakts an der Verbindung gemäß Formel I wird in Beispiel 4 ausführlich beschrieben.

Die Verbindung gemäß Formel I, deren pharmazeutisch annehmbare Salze und Ester, sowie das erfindungsgemäße Rotbuschextrakt sind insbesondere zur Behandlung von Krankheiten des zentralen Nervensystems, bevorzugt Demenzen, Morbus Parkinson, Depressionen, Schmerzzuständen und als Zellschutz-Antioxidans bzw. "Radikalfänger" geeignet. Die Behandlung von Morbus Alzheimer ist besonders bevorzugt.

Erfindungsgemäß können die neuen Verbindungen als Einzelwirkstoff oder in Kombination mit weiteren Wirkstoffen, in der Form gemäß Formel I oder als pharmazeutisch annehmbare Salze oder Komplexe sowie Ester eingesetzt werden. Derivate hiervon hiervon, Salze, Komplexe und Ester sowie deren Herstellung sind dem Fachmann bekannt. Die Herstellung pharmazeutisch annehmbarer Salze ist dem Fachmann ebenfalls bekannt. Als Salzbildner kommen alle üblichen pharmazeutisch annehmbare Säuren bzw. Anionen in Frage. Desweiteren sind Kopplungen an Säuren wie beispielsweise Ferulasäure, Chinasäure, Kaffeesäure, Gluconsäure, Chlorogensäure und verwandte Verbindungen möglich. Insbesondere ist eine Kopplung an Gluconsäure bevorzugt. Die Kopplungsprodukte der Verbindung gemäß Formel I an die vorstehenden Säuren werden als pharmazeutisch verträgliche Derivate bezeichnet. Bevorzugterweise wird die Verbindung aber als Molekül gemäß Formel I verwendet.

Der erfindungsgemäße Rotbuschextrakt sowie die erfindungsgemäße Verbindung gemäß Formel I bzw. deren Salze und Ester, bevorzugt die natürlich vorkommende Verbindung gemäß Formel I, können auf an sich bekannte Weise zu Arzneimitteln und/oder zu Nahrungsergänzungsmitteln mit gesundheitsfördernden Eigenschaften verarbeitet werden. Der erfindungsgemäße Rotbuschextrakt sowie die erfindungsgemäße Verbindung gemäß Formel I bzw. deren Salze und Ester können z.B. in Form von Tabletten, Kapseln, Pillen, Dragees, Granulaten, Pulvern, Lutschpastillen und flüssigen Darreichungsformen wie z.B. Getränken formuliert werden. Bevorzugt ist auch die Verwendung in Nahrungsergänzungsmitteln.

Bevorzugterweise werden die Nahrungsergänzungsmittel bzw. Arzneimittel oral verabreicht, wobei auch beispielsweise die topische, parenterale, intravenöse, intramuskuläre, subkutane, nasale, inhalative, rektale oder transdermale Applikation möglich ist.

Der erfindungsgemäße Rotbuschextrakt sowie die erfindungsgemäßen Arzneimittel bzw. Nahrungsergänzungsmittel können zusätzlich bevorzugt weitere Wirkstoffe, die die Wirkung des Rotbuschextrakts oder der erfindungsgemäßen Verbindung gemäß Formel I bzw. deren Salze verstärken oder die bei den benannten Krankheiten auftretenden Symptome oder Zustände ergänzend positiv beeinflussen (z.B.: weitere Radikalfänger, verschiedene enzymhemmende Stoffe, Vitamine, Lecithine, omega-3-Fettsäuren und Stoffe, die die Funktionen des Gehirns positiv beeinflussen), enthalten.

Desweiteren können pharmazeutisch annehmbare Hilfs- und Trägerstoffe verwendet werden. Geeignete Hilfsstoffe sind dem Fachmann bekannt und umfassen beispielsweise Füllstoffe, Sprengmittel, Gleitmittel, Bindemittel, Benetzungsmittel, etc.

Geeignete Gleitmittel sind z.B. Silikat, Talk, Stearinsäure, Magnesium- oder Calciumstearat und/oder Polyethylenglykole.

Als Bindemittel können beispielsweise Stärken, Gummi arabicum, Gelatine, Methylcellulose, Carboxymethylcellulose oder Polyvinylpyrrolidon verwendet werden.

Aufschlussmittel sind beispielsweise Stärke, Alginsäure, Alginate oder Natriumstärkeglykolate, aufschäumende Mischungen.

Als Benetzungsmittel können beispielsweise Lecithin, Polysorbate oder Laurylsulfate verwendet werden.

Desweiteren können auch Farbstoffe und Süßungsmittel in den Formulierungen enthalten sein.

Die pharmazeutischen Zubereitungen können in bekannter Weise hergestellt werden, z.B. mittels Mischen, Granulieren, Tablettieren, Zuckerbeschichtungs- oder Überzugsbeschichtungsverfahren.

Die flüssigen Dispersionen und/oder Lösungen zur oralen Verabreichung können z.B. Getränke, Tropfen, Sirupe, Emulsionen und Suspensionen sein.

Der Sirup kann als Träger z.B. Saccharose oder Saccharose mit Glycerin und/oder Mannitol und/oder Sorbitol enthalten.

Die Suspensionen und die Emulsionen können als Träger z.B. ein natürliches Harz, Agar, Natriumalginat, Pektin, Methylcellulose, Carboxymethylcellulose oder Polyvinylalkohol enthalten.

Die Suspensionen oder Lösungen für intramuskuläre Injektionen können zusammen mit dem Wirkstoff einen pharmazeutisch annehmbare Träger, z.B. steriles Wasser, Olivenöl, Ethyloleat, Glykole, z.B. Propylenglykol, und, falls gewünscht, eine geeignete Menge an Lidocain-Hydrochlorid enthalten.

Die Lösungen zur intravenösen Injektion oder Infusion können als Träger z.B. steriles Wasser enthalten oder sie können bevorzugt in Form von sterilen, wässrigen, isotonischen Salzlösungen vorliegen.

Die Suppositorien können zusammen mit dem Wirkstoff einen pharmazeutisch annehmbare Träger, z.B. Kakaobutter, Polyethylenglykol, einen Polyoxyethylensorbitol-Fettsäureester oder Lecithin enthalten.

Zusammensetzungen für die topische Applikation, z.B. Cremes, Lotionen oder Pasten, können durch Mischen des Wirkstoffs mit einem herkömmlichen ölhaltigen oder emulgierenden Träger hergestellt werden.

Der erfindungsgemäße Rotbuschextrakt sowie die erfindungsgemäße Verbindung gemäß Formel I bzw. deren Salze und Ester können in üblichen Mengen in den Nahrungsergänzungs- bzw. Arzneimitteln verwendet werden. In Lösung werden bevorzugterweise 0,001 bis 10 Gew.-% der erfindungsgemäßen Verbindung gemäß Formel I bzw. deren Salze, weiter bevorzugt 0,1 bis 7 Gew.-% und besonders bevorzugt 1 bis 5 Gew.-%, eingesetzt.

Der erfindungsgemäße Rotbuschextrakt wird bevorzugt in Mengen eingesetzt, die einer Menge an der Verbindung gemäß Formel I von 1 bis 1000 mg, weiter bevorzugt 10 bis 600 mg, noch weiter bevorzugt 50 bis 400 mg und am meisten bevorzugt 50 bis 250 mg entsprechen.

Die oben beschriebenen Nahrungsergänzungsmittel bzw. Arzneimittel können auf herkömmliche Methoden hergestellt und in einer pharmazeutisch geeigneten Form verabreicht werden.

Die erfindungsgemäß bevorzugten, festen Nahrungsergänzungs- bzw. Arzneimittel können zusätzlich bevorzugt 1 bis 50 Gew.-%, stärker bevorzugt 1 bis 20 Gew.-%, besonders bevorzugt 1 bis 10 Gew.-% Füllstoffe enthalten.

Als Füllstoffe können eine oder mehrere Verbindungen verwendet werden, die einen Teil des Materials zum Erreichen der erforderlichen und gewünschten Tablettengesamtmasse liefern. Einsetzbar sind unter anderem mikrokristalline Cellulose in verschiedenen Partikelgrößen, insbesondere mit einer mittleren Partikelgröße im Bereich von 20 μm bis 200 μm, insbesondere im Bereich von 50 μm bis 150 μm, wie z.B. etwa 100 μm, wie die bekannten Avicel-Produkte wie Avicel PH-101 und PH-102. Weitere geeignete Füllstoffe sind z.B. Maisstärke, Kartoffelstärke, Laktose, Cellaktose (eine Mischung aus Cellulose und Laktose), Calciumphosphat, Dextrose, Mannit, Maltodextrin, Isomalt, gegebenenfalls auch Sorbit und Saccharose. Falls eine Direktverpressung vorgesehen ist, sollte bei der Auswahl der Füllstoffe darauf geachtet werden, dass Qualitäten verwendet werden, die für die Direktverpressung von Tabletten geeignet sind. Dies wird bei den kommerziellen Produkten jeweils vom Hersteller angegeben bzw. kann durch einfache Versuche überprüft werden. Das am stärksten bevorzugte Füllmittel ist mikrokristalline Cellulose (Handelsprodukte sind beispielsweise Avicel, Vivapur und Emcocel).

Geeignete Sprengmittel sind im Stand der Technik bekannt. Sprengmittel werden häufig auch mit dem englischen Begriff "Disintegrants" bezeichnet. Erfindungsgemäß bevorzugte Sprengmittel sind z.B. Crospovidone (Kollidon CL) und Stärke bzw. vorverkleisterte Stärke, insbesondere das Handelsprodukt "Starch 1500". Weitere geeignete Stärken sind z.B. unter den Bezeichnungen Lycatab PGS, Prejel und Sepistab ST 200 kommerziell erhältlich. Weiterhin können auch die bekannten sogenannten "Super Disintegrants" verwendet werden, wie Croscarmellose Natrium (z.B. Ac-Di-Sol, u.a.) und Carboxymethylstärke Natrium (z.B. Explotab, Primojel, u.a.). Besonders bevorzugt sind Stärken wie Starch 1500.

Der Gehalt an Sprengmittel ist in der Regel 1 bis 25 Gew.-%, vorzugsweise 1 bis 20 Gew.-%, insbesondere 2 bis 15 Gew.-%. Geeignete Bereiche für den Gehalt an Sprengmittel sind auch z.B. 2 bis 5 Gew.-% oder 15 bis 20 Gew.-%, in Abhängigkeit der verwendeten Sprengmittel, Füllstoffe und übrigen Zusatzstoffe.

Erfindungsgemäß kann die Zusammensetzung als Gleitmittel eine oder mehrere Verbindungen enthalten, die die Herstellung und die Verarbeitung der Tablette unterstützen. Verwendbare Gleitmittel sind unter anderem Stearinsäure und deren Derivate, wie Calciumstearat, und insbesondere Natriumstearylfumarat (das z.B. unter der Bezeichnung Pruv kommerziell erhältlich ist) und Magnesiumstearat, Glycerolmono-, -di- und insbesondere -tristearat, hydriertes Pflanzenöl (z.B. Lubritab, Dynasan, Sterotext) oder ein Polyethylenglykol (z.B. Lutrol, Carbowax).

Der Gehalt an Gleitmittel ist in der Regel 0,1 bis 4 Gew.-%, vorzugsweise 0,2 bis 4 Gew.-%.

Optional kann die erfindungsgemäße pharmazeutische Zusammensetzung ein oder mehrere Fließregulierungsmittel umfassen. Geeignete Fließregulierungsmittel sind Magnesiumtrisilikat, Talk und insbesondere Siliziumdioxid (z.B. Aerosil). Falls die Zusammensetzung ein Fließregulierungsmittel umfasst, ist dieses in der Regel in einer Menge von 0,5 bis 5 Gew.-%, vorzugsweise 1 bis 4 Gew.-%, insbesondere 2 bis 3 Gew.-% vorhanden.

Die erfindungsgemäßen pharmazeutischen Zusammensetzungen können ebenfalls noch Stabilisatoren für den Wirkstoff enthalten, wie Ascorbinsäure, Zitronensäure, Weinsäure, Milchsäure, etc., bevorzugt Zitronensäure. Der Gehalt an Stabilisator (falls vorhanden) ist in der Regel im Bereich von 0,5 bis 10 Gew.-%, bevorzugt 1 bis 3 Gew.-%.

Die erfindungsgemäßen pharmazeutischen Zusammensetzungen können weitere übliche pharmazeutisch verträgliche Zusatz- und Hilfsstoffe enthalten, bevorzugt enthalten sie aber außer den vorstehend angegebenen (Füllstoff, Sprengmittel, Schmiermittel und gegebenenfalls Fließregulierungsmittel und Stabilisator) keine weiteren Hilfsstoffe.

Einige Füllstoffe wie mikrokristalline Cellulose können auch als Bindemittel dienen. Auch Füllstoffe mit Bindemittelfunktion zählen daher im Rahmen dieser Anmeldung zu den Füllstoffen.

Liegt die erfindungsgemäße pharmazeutische Zusammensetzung als Tablette vor, kann sie mit einem oder mehreren Beschichtungsmitteln filmbeschichtet sein. Verwendbare Beschichtungsmittel sind Hypromellose (Hydroxypropylmethylcellulose), Polyvinylalkohol, Natriumcarboxymethylcellulose und verschiedene Methacrylsäurepolymere (Eudragite), wobei Hypromellose und insbesondere Eudragite bevorzugt sind. Das Beschichten der Tabletten erfolgt auf übliche Art und Weise. In der Beschichtung können außer dem Beschichtungsmittel weitere übliche Bestandteile von Tablettenbeschichtungen wie Weichmacher, Pigmente, Porenbildner oder Suspensionsstabilisatoren vorhanden sein wie z.B. Polyethylenglykol (PEG), Talk oder Titandioxid und gegebenenfalls auch Laktose.

Das Tablettengewicht ist nicht besonders eingeschränkt, üblich sind Tabletten mit 100 mg bis 500 mg bei Verwendung von reinen Wirkstoffen und 500 mg bis 1500 mg bei Verwendung von Extrakten und Pflanzenpulvern. In Kapseln werden Mengen von 100 mg bis 1000 mg verwendet.

Die Dosierungseinheit des Arzneimittels oder Nahrungsergänzungsmittels kann beispielsweise enthalten:

bei peroralen Arzneiformen:

bevorzugt 40 bis 800 mg, besonders bevorzugt 300 bis 600 mg, Rotbuschextrakt pro Tagesdosis. Bei Verwendung der Verbindung gemäß Formel I, sowie deren pharmazeutisch annehmbaren Salze und Ester werden 1/100 bis 1/20 der vorstehenden Mengen eingesetzt. Die Tagesdosis kann beispielsweise in 1 bis 3 Einzeldosen, vorzugsweise in zwei Einzeldosen, täglich verabreicht werden.

bei parenteralen Arzneiformen (zum Beispiel intravenös, subkutan, intramuskulär): bevorzugt 3 bis 60 mg, besonders bevorzugt 10 bis 30 mg, Wirkstoff pro Tagesdosis. Die Tagesdosis kann beispielsweise in 1 bis 3 Einzeldosen, vorzugsweise in einer Einzeldosis, täglich verabreicht werden.

bei Arzneiformen zur rektalen Applikation:

bevorzugt 40 bis 80 mg, besonders bevorzugt 60 mg, Wirkstoff pro Tagesdosis.
Die Tagesdosis kann beispielsweise in 1 bis 3 Einzeldosen, vorzugsweise in einer Einzeldosis, täglich verabreicht werden.

bei Arzneiformen zur Applikation auf die Haut und Schleimhäute (z.B. Lösungen, Lotionen, Emulsionen, Salben usw.):

bevorzugt 40 bis 80 mg Wirkstoff, besonders bevorzugt 60 mg, Wirkstoff pro Einzeldosis.
Die Tagesdosis kann beispielsweise in 1 bis 6 Einzeldosen, vorzugsweise in 1 bis 3 Einzeldosen, täglich verabreicht werden.

Bei Verwendung eines pharmazeutisch annehmbare Salzes muss die verwendete Menge in dem Fachmann bekannter Weise entsprechend angepasst werden.

## Beschreibung der Figuren

**Figur 1** zeigt die Strukturformel von Aspalathin (1) und Catechin(4α->2)-phloroglucinol (2).

**Figur 2** zeigt die Nummerierung der Atome in der Verbindung gemäß Formel I.

**Figur 3** zeigt die Zuordnung der NMR(nuclear magnetic resonance)-Signale zu den Atomen mit den Nummern gemäß Figur 2. Erklärung der Legende: *)/**)/+)/++)/§)/§§)/#)##) = Werte können untereinander ausgetauscht werden, A), B) = Summe des Integrals bzw. chemische Verschiebung der Gruppe nur einmal angegeben.

**Figur 4** zeigt mittels NMR-Experimenten charakterisierte Strukturfragmente A-D der Verbindung gemäß Formel I.

**Figur 5** zeigt Tabelle 2 mit $^{13}C$-NMR-Daten der Verbindung gemäß Formel im Vergleich zu Aspalathin und Catechin(4$\alpha$->2)-phloroglucinol. Aufgrund fehlender $^{13}C$-NMR-Literaturdaten zu Aspalathin erfolgte die Zuordnung der Signale für das bei HWI ANALYTIK GMBH isolierte Aspalathin in Anlehnung an Daten aus: Ho et al., Phytochemistry 1980, 19, 476-477. Die Daten von Catechin(4$\alpha$->2)-phloroglucinol wurden Matthewis et al., J. Agric. Food. Chem. 1997, 45, 1195-1201 entnommen. Erklärung der Legende: *)/**)/+)/++)/§)/§§)/#)##) = Werte können untereinander ausgetauscht werden.

**Figur 6** zeigt den Nachweis der stabilen Versuchsbedingungen bei Verabreichung von Salzlösung an Fischer-344 Ratten nach Verabreichung der Verbindung gemäß Formel I. Auf der y-Achse ist die Zeit in Stunden nach Applikation aufgetragen. Die x-Achse gibt die Frequenzbereiche nach der Fast Fourier Transformation der Daten an: delta, theta, alpha1, alpha2, beta1 und beta2.

**Figur 7** zeigt die Wirkung von 3 mg/kg der Verbindung gemäß Formel I auf EEG Frequenzen bis 5 Stunden nach Applikation. Mittelwerte von n=6 Tieren. Sterne kennzeichnen die statistische Signifikanz, berechnet nach Ahrens und Läuter, 1974. *=p<0.01; **=p<0.05; ***=p<0.025.

**Figur 8** zeigt die Wirkung von 6 mg/kg der Verbindung gemäß Formel I auf EEG Frequenzen bis 5 Stunden nach Applikation. Mittelwerte von n=6 Tieren. Sterne kennzeichnen die statistische Signifikanz, berechnet nach Ahrens und Läuter, 1974. *=p<0.01; **=p<0.05; ***=p<0.025.

**Figur 9** Wirkung der Verbindung der Formel I im Vergleich mit seinen Molekülbestandteilen Aspalathin, Catechin bzw. Epicatechin. Bei etwa äquimolarer Gabe kann die Wirkung auf Grund der einzigartigen Struktur nur von der Verbindung gemäß Formel I, nicht jedoch durch Teile des Moleküls erreicht werden.

**Figur 10** zeigt die Wirkung von 6 mg/kg der Verbindung der Formel I auf EEG Frequenzen während der ersten Stunde nach Applikation. Mittelwerte von n=6 Tieren. Man beachte die Ähnlichkeit zu Medikamenten die zur Behandlung von Demenz, Morbus Parkinson, Depression und Schmerzzuständen eingesetzt werden: L-DOPA (Morbus Parkinson); Tacrin und Galantamin (Morbus Alzheimer; Nefopam (Schmerzbehandlung).

**Figur 11** zeigt das Ergebnis der Motilitätsmessung, die gleichzeitig mit der Messung der Feldpotentiale vorgenommen wurde. Angaben erfolgen in % des Ausgangswertes vor Substanzgabe. Nur (-) Epicatechin verursacht eine deutliche Erhöhung der Motilität.

**Beispiel 1** - Isolierung der Verbindung gemäß Formel I

Zur Herstellung aus der Droge wird unfermentierter grüner Rotbusch vom Hersteller Rooibos Ltd. Clanwilliam Süd-Afrika verwendet.

Als Ausgangsmaterial werden schonend auf einen Feuchtegehalt von kleiner als 10% (besser kleiner als 4%) getrocknete, unfermentierte und zerkleinerte Blätter und/oder Zweigspitzen von Aspalathus linearis verwendet.

Dieses Rohmaterial wird mit einer Mischung aus Methanol und Wasser im Verhältnis 50:50 (Volumenteile) bei 60˚C für 1 Stunde lang unter Rotation extrahiert, wobei das Verhältnis Rohware:Lösemittel 1:7 beträgt. Danach wird die Flüssigkeit von den Pflanzenteilen abfiltriert und die Pflanzenteile erneut auf die gleiche Weise extrahiert und filtriert.

Die beiden Filtrate werden vereinigt und bei vermindertem Druck (220 mbar) und 55˚C vom Methanol befreit. Die verbleibende wässrige Lösung wird mit Wasser zum 5-fachen des Gewichts der eingesetzten Menge getrockneter Pflanzenteile verdünnt und einer Flüssigflüssig-Verteilung unterzogen.

3 Liter der wässrigen Lösung werden 4 mal mit jeweils 1,5 L wassergesättigtem n-Butanol ausgeschüttelt und die vereinigten Butanolphasen unter reduziertem Druck zur Trockne gebracht. Die Ausbeute beträgt ca. 10% der eingesetzten Menge getrockneter Pflanzenteile.

Anschließend erfolgt zunächst eine Grob- und danach eine Feintrennung des Butanolextrakts.

Grobtrennung:

Ca. 50 g Butanolextrakt werden über eine Sephadex LH20-Säule (6 cm Innendurchmesser und 80 cm Füllhöhe = 2260 ml Sephadex LH20) mit 50 Vol% Methanol chromatographiert. Dazu werden die 50 g Butanolextrakt in 400 ml mobiler Phase gelöst und auf die Trennsäule gegeben. Die Säule wird mit einem Fluss von 1,8 ml/Min. so lange gewaschen, bis 3 L Eluat abgetropft sind. Die Säulenfüllung wird nach dem vollständigen Abtropfen der restlichen mobilen Phase entnommen und in 3 L 100%-igem Methanol 10 Minuten lang ausgerührt (extrahiert). Die stationäre Phase wird abfiltriert und das Eluat getrocknet. Der Rückstand beträgt ca. 0,5 bis 1% der eingesetzten Menge Pflanzenteile = Methanolextrakt.

Erste Feintrennung

Ca. 4 g Methanolextrakt werden über eine Sephadex LH20-Säule (3,5 cm Innendurchmesser und 50 cm Füllhöhe = 480 ml Sephadex LH20) mit 80 Vol% Methanol chromatographiert. Dazu werden die 4 g Butanolextrakt in 40 ml mobiler Phase gelöst und auf die Trennsäule gegeben. Die Säule wird mit einem Fluss von 2,4 ml/Min. betrieben und Fraktionen von je 10 Min. Dauer = 24 ml Eluat aufgefangen. Die gesuchte Substanz befindet sich in den Fraktionen Nr. 48-65. Die Ausbeute beträgt bei 4 g Methanolextrakt ca. 0,5 bis 1 g.

Zweite Feintrennung:

Trennsäule: 250 x 30 mm
Stationäre Phase: Reprosil C18 Aqua 10 $\mu$m
Mobile Phase: Methanol 35% (V/V)
Fluss: 1,5 ml/Min.
Fraktionsgröße: 10 Min. = 15 ml
Substanz I befindet sich in den Fraktionen 41 bis 52.
Die vereinigten Fraktionen 41 bis 52 werden lyophilisiert.
Ausbeute ca. 125 mg Substanz I aus 4 g Methanolextrakt.
Chromatographische Reinheit ca. 97% (HPLC).

**Beispiel 2** - Strukturaufklärung der Verbindung gemäß Formel I

Die durch säulenchromatographische Auftrennung erhaltene Verbindung wurde mit den nachfolgenden Geräten charakterisiert und die nachfolgend aufgezählten Messwerte erzielt.

Figur 2 zeigt die Nummerierung der Atome in der erfindungsgemäßen Verbindung gemäß Formel I. In Figur 3 ist Tabelle 1 mit der Zuordnung der NMR-Daten dargestellt.

Die NMR-Daten wurden wie nachfolgend beschrieben durchgeführt und interpretiert.

$^{1}$H-NMR (d6-DMSO (Dimethylsulfoxid))

Das $^{1}$H-NMR der Substanz in DMSO lieferte ein Spektrum, in dem durch Zugabe von deuteriertem Wasser neun austauschende phenolische Protonen sowie fünf aliphatische HO-Protonen durch Signalverkleinerung (H/D-Tausch) identifiziert werden konnten.

Zusätzlich treten acht Protonen im olefinischen Bereich von 5,5 bis 8,0 ppm sowie neun Protonen im für $-CH_xOH/-CH_xOR$-Gruppen (x = 1, 2) typischen Verschiebungsbereich auf. Zwei aliphatische Protonen sind im Bereich um 2,7 ppm identifizierbar, weitere aliphatische Signale (3 Protonen) überlagern mit den $-CH_xOH/-CH_xOR$-Signalen. Ein Teil der Signale tritt verdoppelt bzw. verbreitert auf. Einzelne Integrale entsprechen daher nominell nur einem Isomer, während andere für beide Isomere gemeinsam integriert werden.

$^{13}$C-NMR

Das $^{13}$C-NMR-Spektrum der Substanz zeigt für die Mehrheit der Kohlenstoffatome eine Signalverdopplung. Das Verhältnis der beiden Isomere schwankt abhängig vom verwendeten Lösungsmittel leicht. Es treten elf Signale im Bereich zwischen 25 bis 90 ppm für aliphatische Kohlenstoffatome sowie 24 Signalgruppen im für olefinische Kohlenstoffatome typischen Bereich zwischen 90 und 165 ppm auf. Ein zusätzliches Signal bei 205 ppm wird vom Lösungsmittel überlagert, kann allerdings in d6-DMSO eindeutig identifiziert werden. Aufgrund dieser Überlagerung wurden alle weiteren 2D-Experimente in d6-DMSO durchgeführt.

Die Messung des DEPT(Distortionless Enhancement by Polarization Transfer)-Spektrums erfolgte in d6-Aceton. Das DEPT-Spektrum weist neben drei Methylengruppen eine aliphatische Methingruppe sowie sieben -CHOH-/-CHOR-Signale im Bereich zwischen 65 bis 85 ppm auf. Acht zusätzliche Signale zwischen 95 bis 125 ppm sind olefinischen CH-Gruppen zuzuordnen. Durch Abgleich mit den $^{13}$C-NMR-Signalen kann auf das Vorliegen von 17 quartären Kohlenstoffatomen geschlossen werden.

H/H-Korrelation (COSY(Correlation Spectroscopy))

Das Korrelationsspektrum liefert nur wenige auswertbare Signale, da ein Großteil der Wechselwirkungen im stark überlagerten Bereich von 3,1 bis 3,5 ppm zusammenfallen. Die Signalgruppe H2" bis H4" ist im Bereich von 4,0 bis 4,4 ppm identifizierbar, ebenso ist die Korrelation der Alkylkette H7 (2,7 ppm) bis H8 (3,3 ppm) eindeutig. Ausgehend

von H1' bei 4,7 / 4,8 ppm ist nur eine Korrelation in den Bereich bei 3,1 bis 3,5 identifizierbar.

C/H-Korrelationsspektren (HMQC(Heteronuclear Multiple Quantum Correlation), HMBC(Heteronuclear Multiple Bond Correlation))

Aus den CH-long-range-Korrelationsspektren (HMBC) sind die zu quartären Kohlenstoffatomen benachbarten Protonen sowie auch die Verknüpfung der einzelnen Strukturelemente ableitbar.

Über die Zuordnung der direkten Korrelationen kann ebenfalls geprüft werden, ob die Strukturzuordnung plausibel ist.

Auswertung der erhaltenen Daten und Abgleich mit Literaturwerten

Das Kopplungsmuster der Protonen im Bereich von 6,8 bis 6,4 ppm weist eindeutig auf zwei sich stark ähnliche aromatische Systeme mit 1,3,4-Substitution hin. Anhand der Kopplungen aus den HMBC-Spektren sowie den chemischen Verschiebungen der beteiligten Kohlenstoffatome (145 / 143 / 131 / 119 / 116 / 115 ppm) kann eine 3,4-dihydroxybenzylische Struktur abgeleitet werden. Als Kontaktstellen ist eine Methylengruppe bei 2,7 ppm (30 ppm) - die C7 entspricht - sowie eine -CHOR-Gruppe bei 4,4 ppm (82,5 ppm) - die C2" zugeordnet wird - identifizierbar. Das Fragment mit C7 lässt sich über die zweite Methylengruppe (C8: 3,26 / 46,0 ppm) bis zum Keton an C9 (205 ppm) eindeutig verlängern. Ausgehend von C9 sind dann aber keine weiterführenden Korrelationen erkennbar.

Das C2" enthaltende Fragment weist eine Verknüpfung zu einer weiteren CHOR-Gruppe (4,1 ppm / 71 ppm) auf.

Die Methingruppe bei 4,3 ppm bzw. 37,5 ppm lässt sich über H/H-Korrelationen dem Signal von C4" zuordnen. Von dort sind weitere Ankhüpfungspunkte zu zwei Signalen im Bereich von 103 bis 105 ppm erkennbar die C14 bzw. C10" zugeordnet werden können sowie einer CHOR-Gruppe.

Die Signalgruppe bei 5,7 ppm (2 H, 94 bzw. 96 ppm) zeigt nur kleine meta-Kopplungen (<3 Hz) und kann einem 1,3,5,6-tetrasubstituierten Aromaten mit 3 Sauerstofffunktionen mit weiteren Signalen bei 163 / 161 / 158 und 103 ppm zugeordnet werden. Aufgrund der unterschiedlichen Verschiebung kann eine symmetrische Substitution ausgeschlossen werden.

Die noch nicht zugeordneten sechs -$CH_xOH$ / -CHOR-Gruppen können nicht eindeutig zugeordnet werden, da alle Protonensignale, mehrere direkte und nahezu alle Long-range-Kopplungen in der Signalgruppe um 3,3 ppm zusammenfallen. Aus den chemischen Verschiebungen der Kohlenstoffatome kann jedoch mit guter Sicherheit auf das Vorliegen einer Kohlenhydrat-Seitenkette geschlossen werden.

Die Konnektivität der verbleibenden sechs Signale im Aromatenbereich kann nicht eindeutig geklärt werden, da es sich um sechs quartäre Signale handelt. Diese sind auch über HMBC-Spektren nicht auflösbar.

Damit ergaben sich die folgenden Strukturelemente **A** bis **D** gemäß Figur 4.

Bei näherer Betrachtung lassen sich aus diesen Strukturelementen zwei Naturstoffe konstruieren. So ergibt die Verknüpfung von i mit iii ein in C4"-Position substituiertes Catechin / Epicatechin, während durch Verknüpfung von iv mit vii und von v mit viii ein in Position C14 substituiertes Aspalathinderivat gebildet wird. Beide Substanzen sind aus Rotbuschtee bekannt. Die verbleibenden offenen Verknüpfungen C14 und C4" waren bereits im Verlauf der Elementzuordnung als verknüpft identifiziert worden.

Ein Vergleich der $^{13}C$-NMR-Daten von Aspalathin und einem C4"-substituierten Catechin mit der Verbindung gemäß Formel I (Figur 5) liefert eine gute Übereinstimmung der korrespondierenden Werte. Das Vorliegen von konformativen Isomeren (Atropisomere, Rotationsisomere) kann durch die Behinderung der freien Rotation um die Bindung C14/C4" gut erklärt werden.

Auf eine Gegenüberstellung der $^{1}H$-NMR-Daten wird verzichtet, da durch Signalüberlagerung und Signalverdopplung - die durch das Vorliegen von Rotationsisomeren bedingt ist - ein sehr komplexes Spektrum für die Verbindung der Formel I erhalten wird.

Da die Substanz bislang nicht in der Literatur beschrieben ist, wurde mittels eines Simulationsprogramms (ACD/C+H NMR Predictor V.10.02) die Plausibilität der Zuordnung überprüft. Die Übereinstimmung war bei Vergleich der $^{13}C$-NMR-Daten sehr gut.

Die Zuordnung der drei unbekannten Stereozentren sowie des Kohlenhydrats ist nicht eindeutig möglich, sie erfolgt auf Basis der Strukturanalogie und wird als catchinoide *all* trans-Konfiguration und als β-gluco-Konfiguration zugeordnet.

**Massenspektrum**

Instrument: Micromass Quattro micro API (Fa. Waters); LC-MS

Methode: Electrospray Ionisierung (ESI) / Sekundärionisierung EI

*Ergebnis*

Im ESI-Spektrum tritt ein Molekülradikalkation bei m/z = 741 $[M + H]^+$ sowie ein Clusterion bei m/z = 763 $[M + Na]^+$ auf. Weitere Fragmente treten bei m/z = 453 $[M - Catechin]^+$ und 289 [M - Catechin - Glycosid] auf. Der Substanz ist eine Masse von 740 zuzuordnen.

**UV-Spektrum**

Die Aufnahme des UV-Spektrums der Substanz erfolgte im Rahmen der Prüfung auf chromatographische Reinheit mit HPLC und Dioden-Array-Detektion (DAD) (Trennsystem 1).

*Ergebnis*

Das UV-Spektrum zeigt ein Absorptionsmaximum bei 285 nm und eine Schulter bei 228 nm.

**Beispiel 3** - Tele-Stereo-EEG-Untersuchungen

Die Veränderungen der EEG(Elektroenzephalografie)-Frequenzen wurde nach Gabe von Salzlösung (Kontrolle) bzw. der Verbindung der Formel I oral (3 oder 6 mg/kg Körpergewicht bestimmt.

Die Untersuchungen wurden analog der durch W. Dimpfel beschriebenen Methode (Dimpfel, W., Preclinical data base of pharmaco-specific rat EEG fingerprints (Tele-Stereo-EEG). Eur. J. Med. Res. (2003) 8: 199-207) folgendermaßen durchgeführt:

Sechs männlichen erwachsenen Fischer-344 Ratten (Tag-Nacht konvertiert) wurden im Alter von 6 Monaten 4 bipolar konzentrische Elektroden zusammen mit einem Mikrostecker auf einer gemeinsamen Basisplatte implantiert. Der Stecker diente der Aufnahme eines 4-Kanal-Senders zur telemetrischen Übertragung der aus frontalem Kortex, Hippokampus, Striatum und Formatio Reticularis abgeleiteten Feldpotentiale. Die Signale wurden auf einem Computer System (Software "EEG-Analyse", Betriebssystem OS Science, Laborrechner "LabTeam" der Firma MediSyst, Linden, DE) in Echtzeit einer Fast-Fourier-Transformation unterworfen und die Leistungsdichtespektren jeweils über 60 Minuten gemittelt. Die Unterteilung der Spektren in 6 verschiedene Frequenzbereiche erlaubte die Erfassung pharmako-spezifischer Veränderungen in Bezug auf die jeweils vor Applikation gemessenen Vorwerte innerhalb dieser Frequenzbänder.

Zum Applikationsprotokoll der Substanzen: die Substanzen wurden oral 45 Minuten nach Beginn der Messungen (Vorwert) appliziert. Fünf Minuten später wurden die Messungen wieder gestartet, mindestens über die nächsten 5 Stunden kontinuierlich analysiert und in 60-minütigen Perioden zusammengefasst. Die Testsubstanz wurde in einer Dosierung von 3 und 6 mg/kg (Verbindung der Formel I) appliziert. Die experimentelle Serie wurde mit der Gabe von Salzlösung (Kontrolle), die zu keiner auffälligen Änderung führte, begonnen (Fig. 6).

Der statistische Vergleich der Versuche zu den Ergebnissen, die nach Gabe von Salzlösung gemessen wurde, erfolgte mit Hilfe einer multivariaten Analyse nach Ahrens und Läuter (siehe H. Ahrens, J. Läuter "Mehrdimensionale Varianzanalyse" (1974), Akademie Verlag, Berlin) auf der Basis der Veränderungen innerhalb der einzelnen Frequenzbänder in allen Hirnregionen als Variablen.

Die Verabreichung von Salzlösung führte kaum zu Veränderungen der elektrischen Aktivität ($\mu V^2/\Omega$) im Vergleich zu den Vorphasenwerten (Fig. 6).

Die Verabreichung der Verbindung gemäß Formel I führte zu stabilen Veränderungen der Leistungsdichte in allen Hirngebieten, vor allem während der zweiten und dritten Stunde nach Applikation (siehe Fig. 7 und 8), wobei die Veränderungen sich von denen nach Gabe von Salzlösung trotz der geringen Tierzahl signifikant unterschieden. Bei etwa äquimolarer Gabe von Aspalathin, Catechin oder Epicatechin kann die Wirkung auf Grund der einzigartigen Struktur nur von der Verbindung gemäß Formel I, nicht jedoch durch Teile des Moleküls erreicht werden (Fig. 9). Dies ist auf die besondere Bindungs-Struktur des neu entdeckten Moleküls zurückzuführen. Eine Steigerung der Motilität wurde lediglich in Gegenwart von (-)-Epicatechin beobachtet (Fig. 11) nicht jedoch in Gegenwart der Verbindung gemäß Formel I. Dies betont die eigenständige Wirkung der Verbindung gemäß Formel I.

Die orale Verabreichung der Verbindung gemäß Formel I als Einzeldosis führt zu Veränderungen der elektrischen Hirnaktivität bei den Testtieren, die denen nach Gabe von Metanicotin, Galantamin, Tacrin, L-DOPA, oder Nefopam entsprach. Dieses Muster korreliert in auffälliger Weise mit den Mustern, die für Medikamente, wie sie bereits für die Behandlung von Demenz, Morbus Parkinson und Schmerzzuständen üblicherweise verwendet werden (siehe Fig. 10), nicht jedoch mit Mustern, die bei Medikamenten für andere Indikationen auftreten (Fig. 11).

Die beobachteten Frequenzänderungen wurden mittels einer Diskriminanzanalyse mit den Ergebnissen von Medikamenten zur Behandlung psychiatrischen Erkrankungen des Gehirns wie auch mit Medikamenten für andere

Indikationen verglichen. Beispielhaft können die Wirkstoffe: Galantamin, Nefopam, LSD, Metanicotin, Koffein, Tacrin, Acetylsalicylsäure, Methadon, Metamizol, Fentanyl, Fluvoxamin, Chlorpromazin, Haloperidol, Methohexital, Meprobamat, Midazolam, Valproinsäure und Carbamazepin aufgeführt werden.

Die Ergebnisse einer Diskriminanzanalyse auf der Basis von vier Hirnregionen und 6 Frequenzbändern (24 Variable) zeigten, dass sich die Wirkung der Verbindung gemäß Formel I in die Nachbarschaft von Galantamin, Tacrin sowie Nefopam einordnet.

**Beispiel 4** - Messverfahren zur Bestimmung des Gehalts an Verbindung gemäß Formel I

Der Gehalt der Verbindung nach Formel I in Rotbusch und Zubereitungen aus Rotbusch (Tee, Extrakt, Tabletten) wird mittels HPLC/DAD nach der Methode des externen Standards bestimmt. Zur Gehaltsbestimmung wird die Substanz der Verbindung gemäß Formel I als externer Standard verwendet. Die Auswertung erfolgt bei 280 nm Detektionswellenlänge. Um Oxidationsvorgänge in der Analyselösung zu verhindern, wird den Proben Askorbinsäure zugesetzt.

Das bevorzugt verwendete HPLC-Gerät ist Acquity UPLC/Alliance 2695; Detektor: DAD, 200 bis 400 nm; Säule: Reprosil-Pur ODS-3, 125 x 3 mm, 3 μm, Fa. Dr. Maisch; Säulentemperatur: 60°C.

Eluent: Eluent A: Wasser/Ameisensäure 100/0,2 (V/V);
Eluent B: Acetonitril/Methanol[Wasser/Ameisensäure 50/25/25/0,2 (V/V/V/V).

Injektionsvolumen: 20 μL.
Laufzeit: 95 Min.
Retentionszeit der Verbindung gemäß Formel I: 39,5 Min.

Tabelle 1 (Gradient):

| Zeit (Min.) | Fluss (ml/Min.) | Eluent A % | Eluent B % | Gradientensteigung |
|---|---|---|---|---|
| 0 | 0,3 | 100 | 0 | |
| 40 | 0,3 | 70 | 30 | linear |
| 60 | 0,5 | 20 | 80 | linear |
| 80 | 0,5 | 20 | 80 | linear |
| 81 | 0,3 | 100 | 0 | |
| 95 | 0,3 | 100 | 0 | |

Als Standardlösung wird verwendet:

1 mg der Verbindung gemäß Formel I, genau gewogen, und ca. 20 mg Askorbinsäure werden mit 2 ml Methanol gelöst und mit Wasser zu 20,00 ml aufgefüllt.

Sollkonzentration: 0,05 mg/mL der Verbindung gemäß Formel I und 1 mg/mL Askorbinsäure.

Als Analysenlösung wird verwendet:

Arzneimittelformulierung:

Die zu untersuchende Probe, beispielsweise eine Tablette, wird in einer Pulvermühle pulverisiert und über einem Sieb mit Maschenweite 250 μm gesiebt.

Ca. 0,5 g pulverisierte Probe werden zusammen mit ca. 50 mg Askorbinsäure genau in einen 50 mL Messkolben eingewogen, mit 10 mL Methanol 40°C versetzt und für 10 Min. im Ultraschallbad bei 40°C extrahiert. Anschließend wird mit Wasser bis zur Marke aufgefüllt, kräftig geschüttelt und erneut für 10 Min. im Ultraschallbad bei 40°C extrahiert. Nach dem Erkalten wird gegebenenfalls mit Wasser bis zur Marke aufgefüllt und die Lösung für 5 Min. bei 9300 g zentrifugiert. Der Überstand wird über einen 0,45 μm Membranfilter direkt in ein Braunglasfläschchen für den automatischen Probengeber der HPLC-Anlage filtriert.

Trockenextrakt Auszugsmittel Wasser:

Ca. 125 mg Rotbuschextrakt werden zusammen mit ca. 25 mg Askorbinsäure in einen 25 mL Messkolben

gewogen, mit ca. 22 mL Wasser versetzt, kräftig geschüttelt, gegebenenfalls im Ultraschallbad behandelt und mit Wasser bis zur Marke aufgefüllt.

Trockenextrakt Auszugsmittel nicht Wasser:

Ca. 125 mg Rotbuschextrakt werden zusammen mit ca. 25 mg Askorbinsäure in einen 25 mL Messkolben gewogen, mit ca. 2,5 mL Methanol versetzt und für 10 Min. im Ultraschallbad behandelt. Anschließend wird mit Wasser bis zur Marke aufgefüllt, kräftig geschüttelt und erneut für 10 Min. im Ultraschallbad behandelt.

Auswertung:

Standardlösung und Analyselösung werden direkt hintereinander unter den selben Bedingungen chromatographiert. Die UV-Spektren der Referenzsubstanz werden mit der zur selben Retentionszeit im Analysenchromatogramm detektierten Substanz verglichen und bei Übereinstimmung der Peak als Verbindung gemäß Formel I nach folgender Berechnungsformel berechnet:

$$\text{Gehalt [\%]} = \frac{\text{Peakfläche Analyse} \cdot \text{Verdünnung Analysenlösung (mL)} \cdot \text{Einwaage Standard (mg)} \cdot 100}{\text{Peakfläche Standard} \cdot \text{Verdünnung Standardlösung (mL)} \cdot \text{Einwaage Analyse (mg)}}$$

**Beispiel 5A** - Formulierungen von Monopräparaten der Verbindung gemäß Formel I

Der Ausdruck "Verbindung I" in den Beispielen bezeichnet alle Verbindungen gemäß Formel I sowie deren pharmazeutisch verträgliche Salze und Ester.

Filmtablette:

| | |
|---|---|
| Verbindung I | 50 mg |
| Askorbinsäure | 60 mg |
| Sorbitpulver (Karion instant) | 120 mg |
| Aerosil (hochdisperses Siliciumdioxid) | 3 mg |
| Tablettierhilfsmittel K | 2,8 mg |
| | 235,8 mg |

Film:

| | |
|---|---|
| Schellack | 3,5 mg |
| Ethanol (Lösungsmittel für Schellack) | 97% ca. 70 mg |

Hartgelatinekapsel:

Kapselfüllmasse:

| | |
|---|---|
| Verbindung I | 20 mg |

(fortgesetzt)

| | |
|---|---|
| Askorbinsäure | 60 mg |
| Maltodextrin | 120 mg |
| Aerosil (hochdisperses Siliciumdioxid) | 2 mg |
| Magnesiumstearat | 1,5 mg |

**Beispiel 5B** - Formulierungen von Monopräparaten mit Extrakt enthaltend die Verbindung gemäß Formel I

| | |
|---|---|
| Filmtablette: | |
| Extrakt (mit Verbindung gemäß Formel I) (1%) | 150 mg |
| Askorbinsäure | 60 mg |
| Sorbitpulver (Karion instant) | 80 mg |
| Aerosil (hochdisperses Siliciumdioxid) | 3 mg |
| Magnesiumstearat | 2,5 mg |
| | 295,5 mg |

| | |
|---|---|
| Film: | |
| Schellack | 4,2 mg |
| Ethanol (Lösungsmittel für Schellack) | 97% ca. 84 mg |

| | |
|---|---|
| Hartgelatinekapsel: | |
| Kapselfüllmasse: | |
| Extrakt (mit Verbindung gemäß Formel I) (1%) | 150 mg |
| Askorbinsäure | 40 mg |
| Maltodextrin | 20 mg |
| Aerosil (hochdisperses Siliciumdioxid) | 1 mg |
| Magnesiumstearat | 1,5 mg |
| | 212,5 mg |

## Patentansprüche

**1.** Verbindung der Formel I

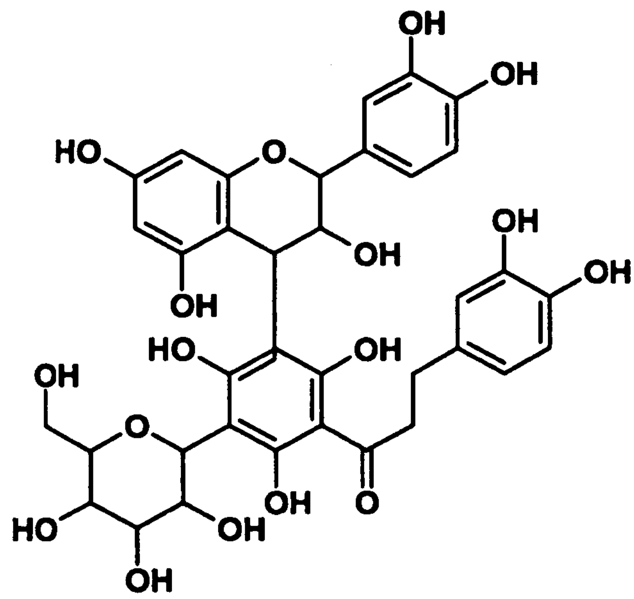

sowie deren pharmazeutisch annehmbare Salze und Ester, erhältlich aus unfermentiertem Rotbusch.

2. Rotbuschextrakt, **dadurch gekennzeichnet, dass** er einen Gehalt an der Verbindung gemäß Formel I von mindestens 0,4 Gew.-% aufweist.

3. Rotbuschextrakt gemäß Anspruch 2, **dadurch gekennzeichnet, dass** der Rotbuschextrakt aus unfermentiertem Rotbusch erhalten wird.

4. Verfahren zur Herstellung der Verbindung der Formel I, **dadurch gekennzeichnet, dass**

- getrocknete und zerkleinerte, unfermentierte Rotbusch-Rohware mit einem Extraktionsmittel bestehend aus Methanol und/oder Wasser für eine vorbestimmte Extraktionsdauer bei einer Temperatur von bis zu 60˚C extrahiert wird,
- der Extrakt filtriert und anschließend unter reduziertem Druck zur Trockne eingeengt wird, und anschließend
- durch drei chromatographische Trennungsschritte unter Verwendung von zwei Sephadex LH20-Säulen und anschließend einer lipophilen c18-HPLC-Säule aufgereinigt wird.

5. Nahrungsergänzungsmittel, **dadurch gekennzeichnet, dass** es Rotbuschextrakt gemäß Anspruch 2 oder 3 in einer Menge von mindestens 100 mg/g Nahrungsergänzungsmittel enthält.

6. Nahrungsergänzungsmittel nach Anspruch 5, **dadurch gekennzeichnet, dass** es mindestens 1 mg der Verbindung der Formel I pro g Nahrungsergänzungsmittel enthält.

7. Verwendung von Verbindungen gemäß Anspruch 1 oder eines Rotbuschextrakts gemäß Anspruch 2 oder 3 zur Herstellung eines Medikaments oder eines Nahrungsergänzungsmittels.

8. Verwendung von Verbindungen gemäß Anspruch 1 oder eines Rotbuschextrakts gemäß Anspruch 2 oder 3 zur Herstellung eines Medikaments zur Behandlung von neurologischen und psychiatrischen Störungen des zentralen Nervensystems.

9. Verwendung gemäß Anspruch 8, worin die neurologischen und psychiatrischen Störungen des zentralen Nervensystems Demenzen, Morbus Parkinson, Depressionen und Schmerzzustände sind.

10. Verwendung gemäß Anspruch 9, worin die Störung des zentralen Nervensystems der Morbus Alzheimer ist.

## Claims

**1.** Compound of the formula I

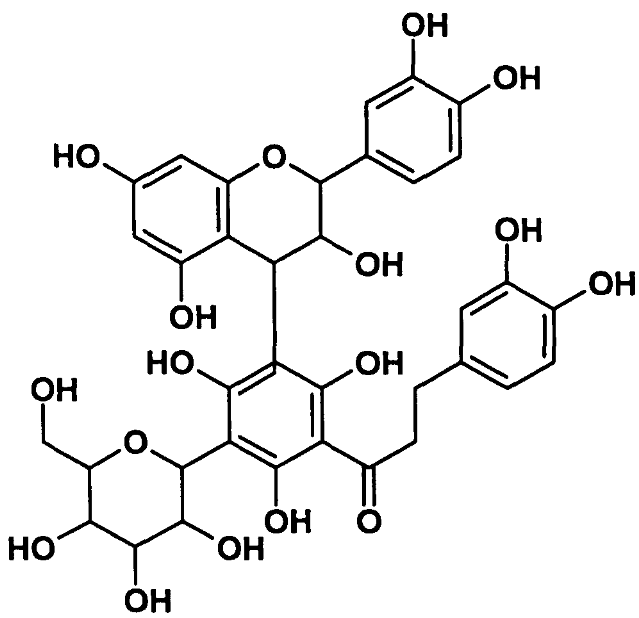

and the pharmaceutically acceptable salts and esters thereof, which can be obtained from unfermented rooibos.

**2.** Rooibos extract, **characterized in that** it has a content of the compound according to formula I of at least 0.4 % by weight.

**3.** Rooibos extract according to Claim 2, **characterized in that** the rooibos extract is obtained from unfermented rooibos.

**4.** Process for the preparation of the compound of the formula I, **characterized in that**

- dried and comminuted, unfermented raw rooibos material is extracted with an extracting agent consisting of methanol and/or water for a predetermined duration of extraction at a temperature of up to 60˚C,
- the extract is filtered and then evaporated to dryness under reduced pressure, and thereafter
- is purified by means of three chromatographic separation steps using two Sephadex LH20 columns and then a lipophilic c18-HPLC column.

**5.** Food supplement, **characterized in that** it contains rooibos extract according to Claim 2 or 3 in an amount of at least 100 mg/g of food supplement.

**6.** Food supplement according to claim 5, **characterized in that** it contains at least 1 mg of the compound of the formula I per g of food supplement.

**7.** Use of compounds according to Claim 1 or of a rooibos extract according to claim 2 or 3 for the preparation of a medicament or a food supplement.

**8.** Use of compounds according to Claim 1 or of a rooibos extract according to Claim 2 or 3 for the preparation of a medicament for treating neurological and psychiatric disorders of the central nervous system.

**9.** Use according to Claim 8, wherein the neurological and psychiatric disorders of the central nervous system are dementias, Parkinson’s disease, depression and pain.

**10.** Use according to Claim 9, wherein the disorder of the central nervous system is Alzheimer's disease.

**Revendications**

**1.** Composé de formule I :

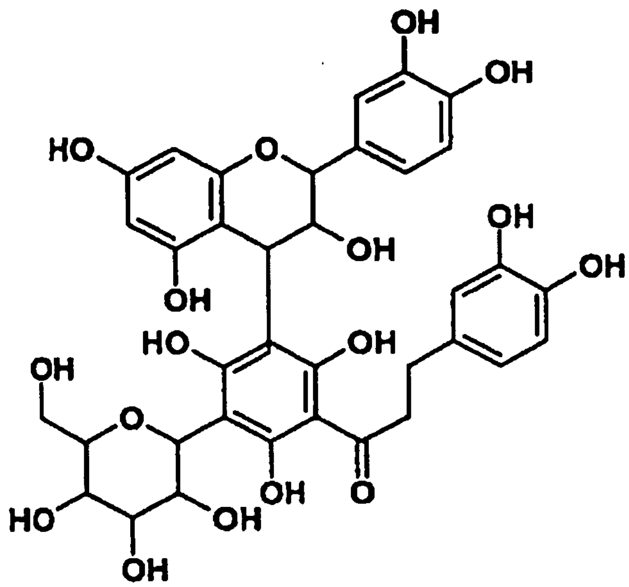

ainsi que ses sels et ses esters acceptables au plan pharmaceutique, que l'on peut obtenir à partir de rooibos non fermenté.

**2.** Extrait de rooibos, **caractérisé en ce qu'**il présente une teneur en composé de formule I d'au moins 0,4 % en poids.

**3.** Extrait de rooibos selon la revendication 2, **caractérisé en ce que** l'extrait de rooibos est obtenu à partir de rooibos non fermenté.

**4.** Procédé pour la fabrication du composé de formule I, **caractérisé en ce que** :

- un produit brut de rooibos non fermenté, séché et broyé, est extrait en utilisant un agent d'extraction constitué de méthanol et/ou d'eau pendant une période de temps d'extraction prédéterminée à une température s'élevant jusqu'à 60 °C,
- l'extrait est filtré, puis concentré sous pression réduite jusqu'à siccité, et ensuite
- purifié par le biais de trois étapes de séparation chromatographique en utilisant deux colonnes Sephadex LH20, puis une colonne HPLC lipophile en C18.

**5.** Complément alimentaire, **caractérisé en ce qu'**il contient un extrait de rooibos selon la revendication 2 ou 3, en quantité d'au moins 100 mg/g de complément alimentaire.

**6.** Complément alimentaire selon la revendication 5, **caractérisé en ce qu'**il contient au moins 1 mg du composé de formule I par gramme de complément alimentaire.

**7.** Utilisation de composés selon la revendication 1 ou d'un extrait de rooibos selon la revendication 2 ou 3, pour la fabrication d'un médicament ou d'un complément alimentaire.

**8.** Utilisation de composés selon la revendication 1 ou d'un extrait de rooibos selon la revendication 2 ou 3, pour la

fabrication d'un médicament destiné à traiter des troubles neurologiques et psychiatriques du système nerveux central.

**9.** Utilisation selon la revendication 8, dans laquelle les troubles neurologiques et psychiatriques du système nerveux central sont représentés par les démences, la maladie de Parkinson, les dépressions et les états de douleur.

**10.** Utilisation selon la revendication 9, dans laquelle le trouble du système nerveux central est la maladie d'Alzheimer.

Figur 1

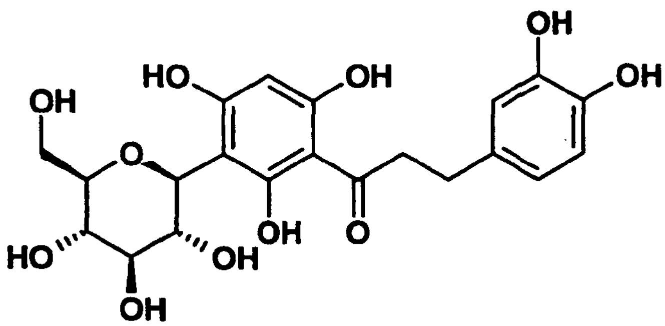

(1)

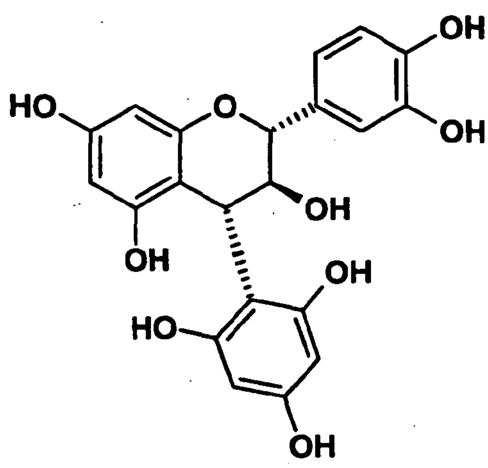

(2)

**Figur 2**

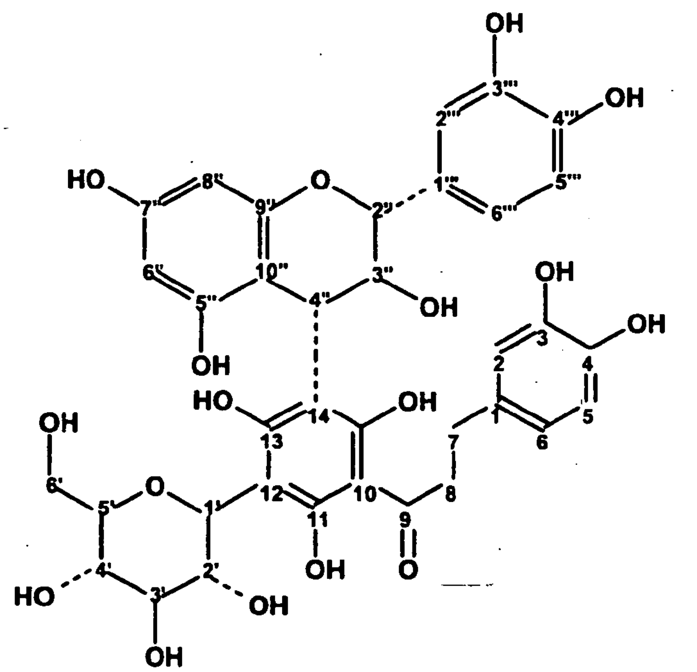

**Figur 3**

Tabelle 1

| | $^{13}$C-NMR (δ in ppm) | $^{1}$H-NMR [δ in ppm, Integral, Multiplizität (J in Hz)] |
|---|---|---|
| **C1** | 130,7 /130,6[+)] | - |
| **C2** | 115,6[§§)] | 6,46 (4 H, m)[A)] |
| **C3** | 143,3[#)] | - |
| **C4** | 145,0[#)] | - |
| **C5** | 115,0[§§)] | A) |
| **C6** | 119,0[§)] | 6,46 (1 H, m) |
| **C7** | 29,8 | 2,73 (2 H, t, 7,7) / 2,67 (2 H, t, 7,3) |
| **C8** | 46,1 / 45,9 | 3,26 (2 H, m) |
| **C9** | 205,1 | - |
| **C10** | 110,7 / 110,7[++)] | - |
| **C11** | 155,9 / 155,8[##)] | - |
| **C12** | 105,2 / 104,9[++)] | - |
| **C13** | 156,2[##)] | - |
| **C14** | 104,8 / 104,0[++)] | - |
| **C15** | 157,2[##)] | - |
| **C1'** | 76,0 / 75,5 | 4,83 (d, 9,8) / 4,68 (d, 9,6) |
| **C2'** | 81,4 / 81,2[*)] | 3,29 (4 H, m )[*)B)] |
| **C3'** | 77,8[*)] | B) |
| **C4'** | 73,7 / 73,2[*)] | B) |
| **C5'** | 69,1 / 68,9[*)] | B) |
| **C6'** | 60,0 / 59,8 | 3,61 (2 H, m) |
| **C2"** | 82,5 | 4,41 (1 H, d, 8,5) / 4,32 (1 H, d, 8,2) |
| **C3"** | 71,1 / 70,9 | 4,10 (1 H, m) |
| **C4"** | 37,6 / 37,4 | 4,30 (1 H, d, 9,7) / 4,26 (1 H, d, 9,8) |
| **C5"** | 158,3 / 157,5[##)] | - |
| **C6"** | 94,5 / 94,4[**)] | 5,66 (1 H, "t", 2,5)[**)] |
| **C7"** | 161,9 / 160,0[##)] | - |
| **C8"** | 96,3 / 69,1[**)] | 5,7 , (1 H, "t", 2,6)[**)] |
| **C9"** | 163,0[##)] | - |
| **C10"** | 103,6 / 103,4[++)] | - |
| **C1'''** | 132,6 / 132,6[+)] | - |
| **C2'''** | 115,9 / 115,8[§§)] | A) |
| **C3'''** | 144,8 / 144,7[#)] | - |
| **C4'''** | 145,0[#)] | - |
| **C5'''** | 115,4 / 115,1[§§)] | 6,81 (1 H, d, br)[§§)] |
| **C6'''** | 119,5 / 119,3[§)] | A) |

Figur 4

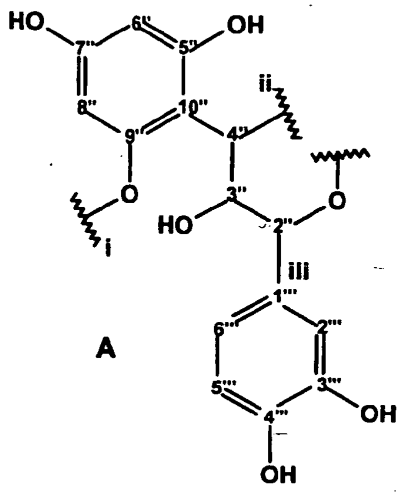

A

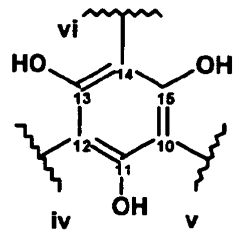

B

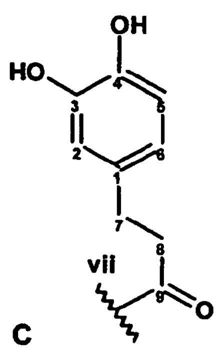

C

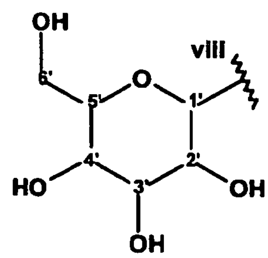

D

**Figur 5**

Tabelle 2

| | **Verbindung gemäß Formel I** (δ in ppm, DMSO) | **Aspalathin** (δ in ppm, DMSO) | **Catechin(4α->2)-phloroglucinol** (δ in ppm, Aceton) |
|---|---|---|---|
| **C1** | 130,7 / 130,6[+)] | 132,5 | |
| **C2 / C5** | 115,6[§§)] // 115,0[§§)] | 115,8 // 115,6 | |
| **C3 / C4** | 145,0[#)] // 143,3[#)] | 145,0 / 143,3 | |
| **C6** | 119,0[§)] | 118,9 | |
| **C7** | 29,8 | 29,8 | |
| **C8** | 46,1 / 45,9 | 45,5 | |
| **C9** | 205,1 | 204,5 | |
| **C10** | 110,7 / 110,7[++)] | 104,1 | |
| **C11** | 155,9 / 155,8[##)] | 164,7 | |
| **C12** | 105,2 / 104,9[++)] | 103,8 | |
| **C13** | 156,2[##)] | 163,6 | |
| **C14** | 104,8 / 104,0[++)] | 94,8 | |
| **C15** | 157,2[##)] | 161,7 | |
| **C1'** | 76,0 / 75,5 | 73,8 | |
| **C2'** | 81,4 / 81,2[*)] | 81,3 | |
| **C3'** | 77,8[*)] | 79,0 | |
| **C4' / C5'** | 73,7 / 73,2[*)] // 69,1 / 68,9[*)] | 70,9 / 70,5 | |
| **C6'** | 60,0 / 59,8 | 61,3 | |
| **C2"** | 82,5 | | 82,2 |
| **C3"** | 71,1 / 70,9 | | 70,8 |
| **C4"** | 37,6 / 37,4 | | 36,4 |
| **C5" / C7" / C9"** | 158,3 / 157,5[##)] // 161,9 / 160,0[##)] // 163,0[##)] | | 156,8 - 155,6 (3x C) |
| **C6" / C8"** | 96,3 / 96,1[**)] // 94,5 / 94,4[**)] | | 95,5 (2x) |
| **C10"** | 103,6 / 103,4[++)] | | 104,0 |
| **C1'''** | 132,6 / 132,6[+)] | | 130,7 |
| **C2'''** | 115,9 / 115,8[§§)] | | 113,8 |
| **C3''' / C4'''** | 144,8 / 144,7[#)] // 145,0[#)] | | 143,6 / 143,5 |
| **C5'''** | 115,4 / 115,1[§§)] | | 114,1 |
| **C6'''** | 119,5 / 119,3[§)] | | 119,0 |

Figur 6

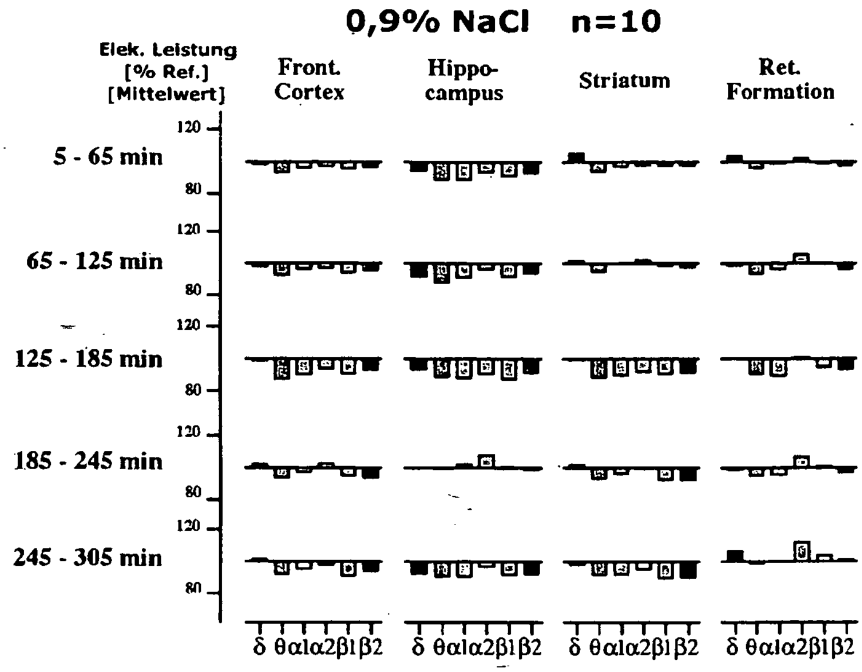


Figur 7

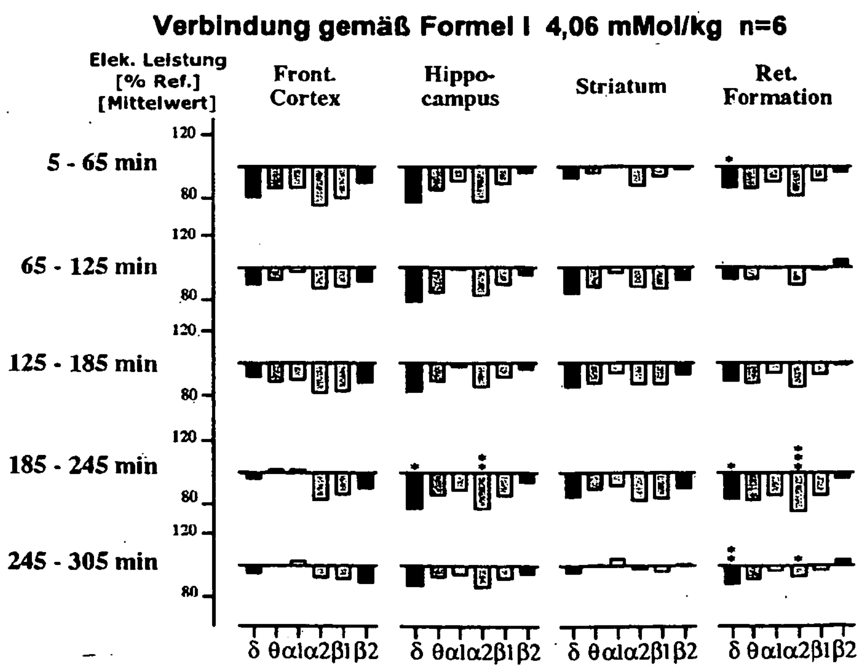

**Figur 8**

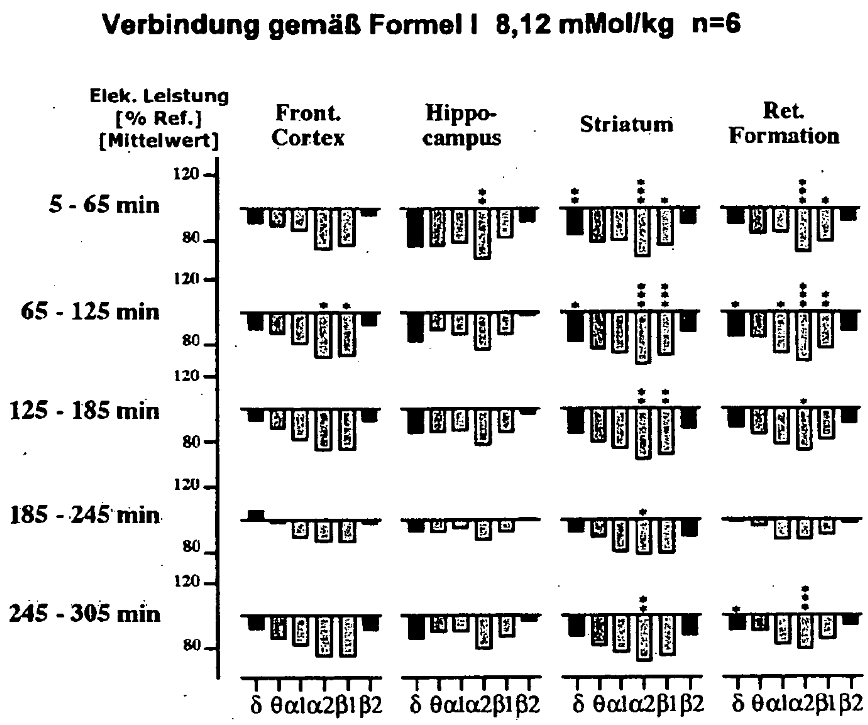


**Figur 9**

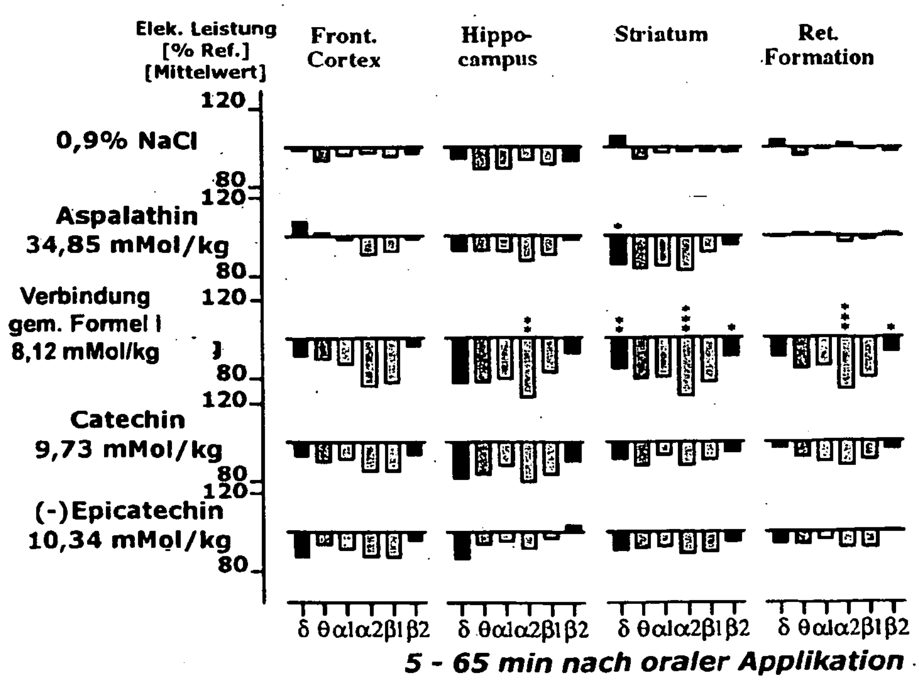

Figur 10

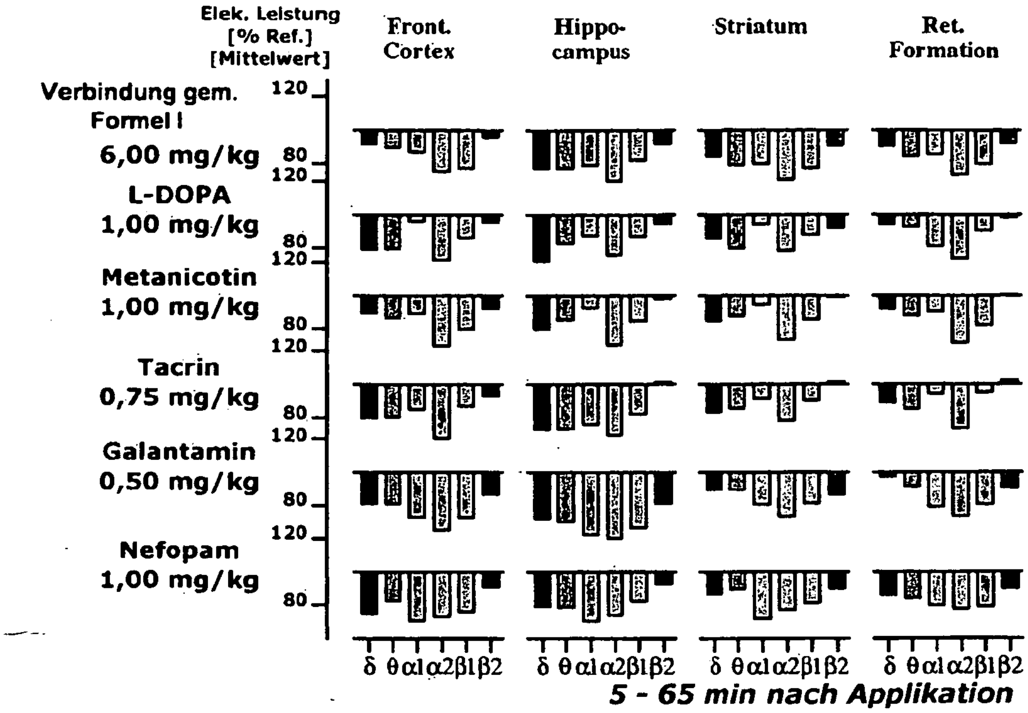


Figur 11

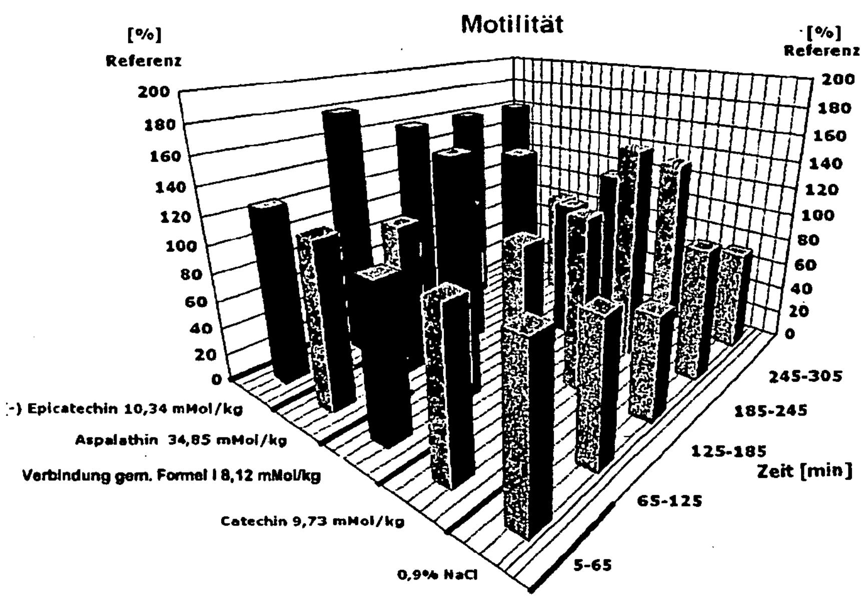

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102005004438


### In der Beschreibung aufgeführte Nicht-Patentliteratur


- **BRAMATI et al.** *J. Agric. Food Chem.,* 2003, vol. 51, 7472-7474
- **SCHULZ et al.** *Eur. Food Res. Technol.,* 2003, vol. 216, 539-543
- **HO et al.** *Phytochemistry,* 1980, vol. 19, 476-477
- **MATTHEWIS et al.** *J. Agric. Food. Chem.,* 1997, vol. 45, 1195-1201
- **DIMPFEL, W.** Preclinical data base of pharmaco-specific rat EEG fingerprints (Tele-Stereo-EEG). *Eur. J. Med. Res.,* 2003, vol. 8, 199-207